Europäisches Patentamt

(19) European Patent Office (11) Publication number: **0 078 314**

Office européen des brevets **B1**

(12) **EUROPEAN PATENT SPECIFICATION**

(45) Date of publication of patent specification: **14.01.87** (51) Int. Cl.⁴: **A 61 F 2/06,** A 61 L 27/00

(21) Application number: **82901886.0**

(22) Date of filing: **05.05.82**

(86) International application number:
**PCT/US82/00594**

(87) International publication number:
**WO 82/03764 11.11.82 Gazette 82/27**

(54) FABRICATION OF LIVING BLOOD VESSELS AND GLANDULAR TISSUES.

(30) Priority: **08.05.81 US 261928**
**26.02.82 US 352585**

(43) Date of publication of application:
**11.05.83 Bulletin 83/19**

(45) Publication of the grant of the patent:
**14.01.87 Bulletin 87/03**

(84) Designated Contracting States:
**AT BE CH DE FR GB LI NL SE**

(56) References cited:
**EP-A-0 002 931**
**AU-B- 516 741**
**DE-A-2 017 330**
**GB-A-1 510 163**
**US-A-3 425 418**
**US-A-3 625 198**
**US-A-3 883 393**
**US-A-4 254 226**
**US-A-4 317 886**

**Bell, E. Ivarsson, B., and Merrill, C., Proc. Natt. Acad. Sci. U.S.A., vol. 76, no. 3, pp. 1274-1278, Cell. Biology, March 1979**

(73) Proprietor: **MASSACHUSETTS INSTITUTE OF TECHNOLOGY**
**77 Massachusetts Avenue**
**Cambridge, MA 02139 (US)**

(72) Inventor: **BELL, Eugene**
**1150 High Street**
**Dedham, MA 02026 (US)**

(74) Representative: **Holdcroft, James Gerald, Dr. et al**
**Graham Watt & Co. Riverhead**
**Sevenoaks Kent TN13 2BN (GB)**

## Description

This invention is in the field of biology and particularly relates to the fabrication of living tissue in tubular form for various applications such as capillaries, larger blood vessels and glandular prosthesis.

An article in *Proc. Natl. Acad. Sci. USA* Vol. 76 No. 3 pp 1274—1278 March 79 entitled "Production of a Tissue-Like Structure by Contraction of Collagen Lattices by Human Fibroblasts of Different Proliferative Potential In Vitro" by Bell et al discloses the fabrication of planar, skin-like living tissue. This living tissue is produced *in vitro* by forming a hydrated collagen lattice, containing a contractile agent comprising fibroblast cells which contract the lattice. This skin-like tissue is formed in a round or rectangular vessel with, or without, a frame of stainless steel mesh lying on the floor of the vessel. In its absence, the lattice contracts in all dimensions; in its presence as the lattice sets it becomes anchored to the mesh and contracts in the thickness dimension only. The mesh, resembling a picture frame, holds the lattice of living tissue within it. The contracted lattice, with or without the stainless steel mesh frame, can be seeded with epidermal cells from the potential graft recipient. When a sheet of epidermal cells forms, the two layered skin equivalent is grafted.

The resultant graft is unique as compared to any other graft obtained from artificial skin since its basic organization is like that of skin and its living constituent cells are donated by potential graft recipients.

This invention extends and applies the teaching of the aforesaid article in Proc. Natl. Acad. Sci. USA to the preparation of tubular prostheses suitable for *in vivo* use as blood vessels and capillary beds, for instance.

US—A—3,425,418 from which this invention commences discloses the *in vitro* preparation of artificial blood vessels by casting hydrated collagen about a core and thereafter dehydrating the collagen. The core comprises a woven tube which is embedded in the collagen matrix. Dehydration is achieved, contracting the collagen, by immersing the casting in a solvent mixture. Other methods of dehydration that are disclosed include air drying and the use of tanning agents. There is no disclosure of the use of cellular contractile agents to contract the collagen.

According to the present invention there is provided a method of producing, *in vitro* a laminated collagenic tubular structure, suitable for *in vivo* use as artificial vessels and glandular prostheses, by casting collagen about a core, characterised by:

a. producing a first tubular layer by the steps of:

(1) forming a first aqueous mixture of collagen fibrils, nutrient medium and a first cellular contractile agent capable of interacting with collagen fibrils,

(2) introducing the said first mixture into an annular casting chamber,

(3) maintaining the annular casting chamber containing the said first mixture under conditions sufficient to allow a gel to form therein and to allow radial contraction of the gel with expression of aqueous liquid therefrom resulting from interaction of the cellular contractile agent with collagen fibrils thereby to form a contracted hydrated collagen lattice as the first layer of the multi-layered tubular structure, and

(4) removing aqueous liquid expressed in the formation of the first layer from the annular casting chamber;

b. producing a second tubular layer outwardly of the first layer by the steps of:

(1) forming a second aqueous mixture of collagen fibrils, nutrient medium and a second cellular contractile agent capable of interacting with collagen fibrils,

(2) introducing the said second mixture into the annular casting chamber,

(3) maintaining the annular casting chamber containing the said second mixture under conditions sufficient to allow a gel to form therein and to allow radial contraction of the gel with expression of aqueous liquid therefrom resulting from interaction of the cellular contractile agent with collagen fibrils to thereby form a second contracted hydrated collagen lattice as another layer of the multi-layered tubular structure; and

c. removing the resulting living multi-layered tubular structure from the annular casting chamber.

Also according to the present invention, there is provided a method of producing, *in vitro* a laminated collagenic tubular prosthesis, suitable for *in vivo* use, by casting collagen about a core, characterised by:

a. fabricating a cylindrical smooth muscle cell layer as follows:

(aa) separately preparing (i) an aqueous acidic mixture comprising nutrient medium and collagen fibrils and (ii) a mixture of smooth muscle cells suspended in nutrient medium,

(ab) raising the pH of mixture (i) to neutrality and quickly combining mixture (i) and mixture (ii) and pouring the combined mixture into a casting chamber having an inner core member and an outer cylindrical wall structure, to form a first lattice,

(ac) incubating the first lattice for a period sufficient to enable collagen fibrils to be compacted by the cells so that aqueous liquid is expressed out of the lattice as the lattice contracts radially about the core,

(ad) removing aqueous liquid expressed in step (ac),

(ae) repeating steps (aa)—(ad) if additional layers of smooth muscle cells are desired;

b. fabricating a second layer, containing fibroblast cells, on said cylindrical smooth muscle layer(s) as follows:

(ba) separately preparing (i) an aqueous acidic mixture comprising nutrient medium and collagen fibrils and (ii) a mixture of fibroblast cells suspended in nutrient medium,

(bb) raising the pH of mixture (i) to neutrality and quickly combining mixture (i) and mixture (ii) and pouring the combined mixture into a casting chamber having as an inner core member the previously-formed cylindrical smooth muscle cell layer and an outer cylindrical wall structure, to form a second lattice,

(bc) incubating the second lattice for a period sufficient to enable collagen fibrils to be contracted by the fibroblast cells so that aqueous liquid is expressed out of the second lattice as it contracts radially about the smooth muscle layer(s) disposed internally of the second lattice, and

(bd) removing aqueous liquid expressed in step (bc); and

c. lining the inner wall of the cylindrical smooth muscle cell layer with living cells.

The invention further provides a method of producing, *in vitro* a collagenic structure by casting hydrated collagen about core means to form a pierced collagenic structure, suitable for *in vitro* use as a capillary bed, characterised by the steps of:

a. forming a mixture of collagen fibrils, nutrient medium and a cellular contractile agent capable of interacting with collagen fibrils to produce a contracted collagen structure;

b. introducing the said mixture into a casting chamber containing a plurality of threads therein, said threads serving as core means for defining a plurality of capillary passages in the finished collagenic structure;

c. maintaining the casting chamber under conditions which allow a gel to form around the threads and the allow radial contraction of the gel resulting from interaction of the cellular contractile agent with collagen fibrils, thereby to form a contracted hydrated collagen lattice having a plurality of threads embedded therein;

d. removing the threads from the lattice to produce a plurality of capillary channels throughout the contracted collagenic structure; and

e. lining the capillary channels on their inner surfaces with endothelial cells.

The cast collagen lattices contracted by living cells, such as fibroblasts, smooth muscle cells, or elements of cells such as blood platelets are cast into shapes which provide internal surface areas and tubular shaped terminals, or end structures, particularly effective for making connections, *in vivo*, with existing tubular structures, such as capillaries, blood vessels and glandular tissues.

The internal surface of the case structure can be lined with specialized cells, depending on the function of the structure. For example, endothelial cells are used for the internal surface of an artery, vein, or other structures with internal surfaces.

Alternatively, in some applications it may be desirable to line the internal surface with specialized cells having a predetermined therapeutic value. For example, the inner surfaces of a capillary bed may be lined with pancreatic β cells to boost the insulin supply in the blood. Pancreatic islets (islets of Langerhans), hepatocytes or other types of glandular cells may also be used for lining the inner surface of the vessel-equivalent structures.

In one embodiment, the central core for forming the tube consists of polyethelene or glass tubing. This core is axially centered within a cylindrical mold. Suitable tissue forming constituents are poured into the cylindrical mold. After a suitable period of time, the tissue forming constituents contract the lattice and close in around the central core. This procedure can be repeated as many times as desired with the same or different cell types in the same or different proportions to yield a multilayer tube. After each layer contracts the fluid expressed from the contracting lattice is poured off to accommodate the tissue forming constituents of the next layer. The central core may then be removed and suitable cells, predicated on the function of the cast structure, may then be cultured on the inner surface of the hollow tissue cylinders, to form, for example, a vessel-equivalent structure.

The fortuitous fact that the lattice contracts radially about the central core structure to form tubes enables one to form various shaped structures defined by the inner core surface. If, instead, the lattice contracted in all directions, the resultant structure would end up as a shapeless mass at the bottom of the mold. It is also important to note that in the formation of vessel-equivalent structure, in accordance with the invention, the sequential addition of cells in an ordered pattern of layers is essential.

The vessel-equivalent structure thus far described is devoid of elastin, the fibrous mucoprotein which is the major connective tissue protein of elastic structures (e.g. large blood vessels). Without this elastic property it is possible that the vessel could burst under pressure. Since elastin is an extremely insoluble substance it is difficult to directly incorporate elastin into the molded tissue forming constituents previously described. Accordingly, a plastic mesh may be optionally provided between two layers or within a layer of the tissue forming constituents during the molding process, as will be described in detail.

This mesh serves to reinforce the resultant vessel and at the same time provide a degree of elasticity to the structure so that it may expand and contract in the manner of a natural blood vessel having elastin.

Brief Description of the Drawings

Fig. 1 is a perspective view of a first embodiment of the invention showing the structure of the casting chamber.

Fig. 1A is a cross-sectional view of Fig. 1 showing a vessel as cast.

Fig. 1B is a perspective view showing a plastic mesh on a support tube which is used to position the mesh during casting.

Fig. 2 is a schematized view showing the culturing apparatus of the invention.

Fig. 3 is a top view of an embodiment suitable for producing a plurality of connecting elements, such as capillaries, within a lattice structure.

Fig. 4 is a side-section showing the mold used in connection with Fig. 3.

Fig. 5 is a top view of a further embodiment of the invention.

Best Mode of Carrying Out the Invention

The following description relates to the casting of cylindrical structures intended as prosthesis for vessels or capillaries since such structures are commonly found in the human body. However, other shapes may be conveniently cast in accordance with the teachings herein and the invention is not intended to be limited to any particular shape or body structure.

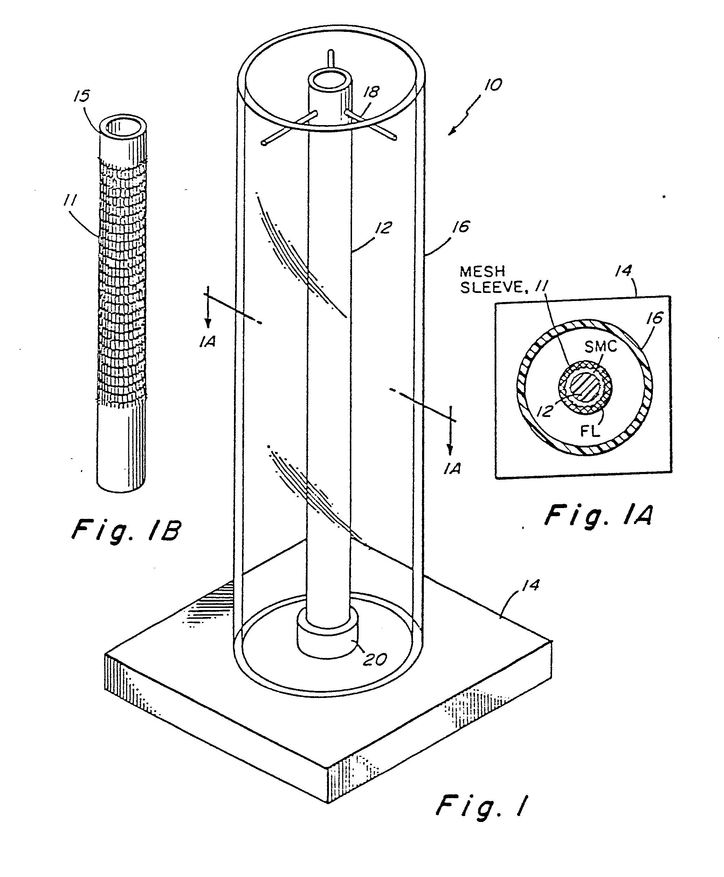

Fig. 1 shows a preferred form of casting chamber for fabricating a blood vessel-equivalent of living matter. The casting chamber 10 comprises a central rod or mandrel 12 disposed in a cylinder 16. The central rod and cylinder are mounted on a base or stand 14. The rod 12 is provided with three arms or spokes 18 at the top of the rod for centering the rod within the cylinder 16.

The base is provided with an appropriate collar 20 to accept the central rod 12. The outer cylinder has an internal diameter such that when the arms 18 are disposed as shown and the central rod is located in the collar 20, the rod 12 will be centered within cylinder 16. The outer diameter of the rod 12 determines the inner diameter of the cast vessel and for many applications would be in the range of from 2—10 mm.

With the diameter of the central rod kept constant, the inner diameter of cylinder 16 will determine the final thickness of the cast layer, and typically may range from 1—4 cm to produce a final thickness of about 0.5—2 mm, the final thickness being proportional to the diameter. The height of the chamber determines the length of the vessel and would typically be between 10—30 cm in height.

The casting chamber parts should be made from material which may be readily cleaned and is autoclavable. Preferably, the cylinder 16 should be made from material which is clear and which will permit diffusion of carbon dioxide and other gases. Thus, the rod 12 may be made of glass or metal and the cylinder 16 should preferably be made of autoclavable plastic, such as polycarbonate. The stand 14 may be made of glass, plastic or metal, such as stainless steel.

The size and structure of blood vessels varies in accordance with the function of the particular blood vessel. Blood vessels may be generally characterized by their cellular composition and the composition of the matrix or collagen lattice with which other extracellular elements, such as elastin fibers and proteoglycans are associated. The collagen, elastin, and proteoglycans are the biosynthetic products of the cells in each of the layers.

The cell types are endothelial, smooth muscle, and fibroblasts (called pericytes) and are found respectively in successive layers from the lumen outward. In order to construct a particular type of blood vessel, the respective layers may be laid down in order. Alternatively, several can be laid down concurrently. All vessels contain an inner endothelial lining. In an artery, for example, smooth muscle surrounds the endothelium and the final outside layer is made up of fibroblasts.

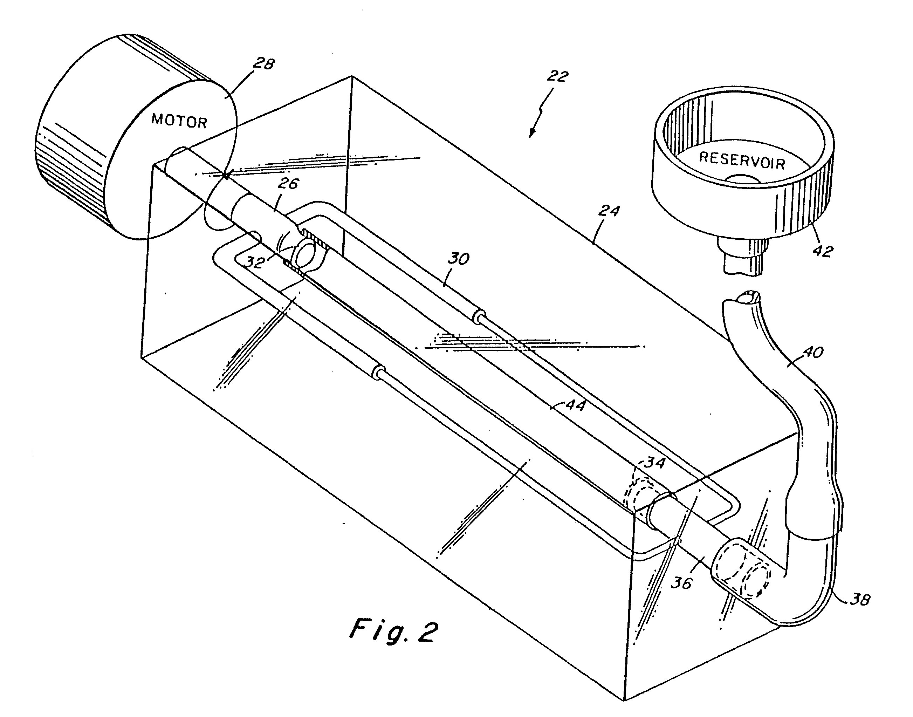

The process for fabricating the above described multilayered blood vessel-equivalent will now be described in detail in connection with Figs. 1 and 2.

First, the smooth muscle layer is fabricated. A mixture of nutrient medium (e.g. McCoy's medium containing fetal bovine serum) is prepared in a flask. The ingredients are mixed in the following ratio: 9.2 ml of $1.76 \times$ concentrate of McCoy's medium and 1.8 ml of fetal bovine serum. The pH is raised by addition of 1.0 ml of 0.1N NaOH. The foregoing mixture of medium and serum is poured onto a dish in which 1.5 ml of native collagen in a 1—1000 acidic acid solution has been prepared. About 250,000 cultured aorta smooth muscle cells suspended in a 0.5 ml of McCoy's medium supplemented with a 10% fetal bovine serum is quickly added. The above constituents are mixed by swirling the dish and quickly pouring the mixture into the casting chamber. The chamber is then placed in a humidified 5% $CO_2$, 95% air incubator at 37°C for 3 days.

A collagen lattice or gel forms immediately on casting the mixture. The collagen fibrils are gradually compacted by the cells so that fluid is squeezed out of the lattice. The result is contraction of the collagen lattice around the central core or rod 12. After 3 days in the incubator, the smooth muscle layer will have set in a cylindrical structure having sufficient structural integrity to simulate, or replicate, the smooth muscle layer of a typical blood vessel. If a second layer is to be applied, the fluid expressed during contraction of the first lattice is poured off and a second complete mixture of all ingredients is added to replace the said fluid, and another contracted layer is deposited on the first formed layer. The process may be repeated as many times as desired to give a multilayered structure. The layers may be poured simultaneously with a removable separation or sleeve (not shown) between them. As soon as gelation begins the sleeve is removed.

Optionally, after the smooth muscle layer cylinder (SMC) has been cast, it may be desirable to provide a plastic mesh sleeve 11 about the outer surface of the smooth muscle layer cylinder or the mesh may be embedded in the smooth muscle layer. This mesh will serve to reinforce the resulting structure and provide some degree of elasticity so that the resulting structure will be better able to withstand the pressures it will be subjected to in use. Meadox Medicals, Inc., 103 Bauer Drive, Oakland, New Jersey 07436, supplies a Dacron® mesh sleeve, Part No. 01H183, which has proved particularly suitable for this purpose. Other suitable meshes are readily available in various inert plastics, such as Teflon®, nylon, etc. and the invention is not to be limited to a particular plastic material. Preferably, the mesh should be treated to render it more electronegative by, for example, subjecting it to plasma. This results in better cell attachment to the plastic sleeve and hence an increase in the strength of the resultant structure.

The sleeve 11 should be placed on the smooth muscle cell cylinder by first disposing the sleeve 11 on metal tube 15 (as shown in Fig. 1B) which has an inner diameter larger than the outer diameter of the smooth muscle cell cylinder. The tube 15, with the sleeve on the exterior, is then slipped over the smooth muscle cell cylinder, a portion of the sleeve is then pulled off the tube 15 and onto the smooth muscle cell cylinder and held there while the tube 15 is slipped off the smooth muscle cell cylinder. This procedure minimizes damage to the exterior surfaces of the smooth muscle cell cylinder while attaching the sleeve.

Next, a fibroblast layer (FL) may be cast around the inner smooth muscle layer(s) (SMC) and sleeve 11 so as to completely enclose the sleeve 11, as shown in Fig. 1A. In this process, the ingredients described above in connection with the fabrication of a smooth muscle layer are used to constitute a fibroblast layer, except that cultured aorta fibroblasts are substituted for the smooth muscle cells. The incubation period for the fibroblast layer may be 2 days to a week.

The resultant multi-layered structure consisting of inner smooth muscle layer(s) and an outer fibroblast layer with a mesh sleeve sandwiched between the two layers is now ready to be cultured with an inner endothelial lining of living endothelial cells. To perform this step the cylindrical tissue tube of several layers is slipped off the casting rod 12 to receive the endothelial cells as a suspension. It is supported in the culturing apparatus shown in Fig. 2.

The apparatus of Fig. 2 comprises a transparent chamber 24, within which a rotatable rod 26 is inserted at one end and a rotatable tube 36 is inserted at the opposite end. The tube 36 and rod 26 are tied together by wire frame member 30 such that when the rod 26 is rotated, the tube 36 will rotate in unison in the same direction. Rod 26 is coupled to motor 28 such that when motor 28 is energized the rod 26 will rotate in the direction shown by the arrow. Preferably, the rod is attached to the motor in such a way that the length of the rod inserted into the chamber 24 may be adjusted in accordance with the length of the vessel-equivalent 44 being supported within the culture chamber 24. This may accomplished by a rack and pinion device or other such variable length means (not shown).

Rod 26 is provided at one end with a nipple 32 to which a vessel 44 (such as the structure previously described in connection with Figs. 1, 1A and 1B comprising an inner cylinder smooth muscle cell layer, and an outer cylinder of fibroblast cells with a mesh sleeve sandwiched between) may be attached. Similarly, tube 36 is provided with a complementary nipple 34 to which the opposite end of the vessel 44 may be attached. In this manner, the vessel 44 is suspended between the rod 26 and tube 36 and a culture medium may be introduced from reservoir 42 through tubing 40 and fixed connecting tube 38, through tube 36 and into the interior lining of blood vessel-equivalent 44. It should be understood that watertight seal bearings (not shown) are provided at both ends of chamber 24 to permit the rod and tube to be inserted into the chamber.

Reservoir 42 is supplied with a suspension of about 200,000 cultured aorta or other endothelial cells in McCoy's medium supplemented with a 20% fetal bovine serum. This mixture is fed by hydrostatic pressure from the reservoir into the vessel 44 as previously mentioned. Next, the vessel 44 is slowly rotated by means of motor 28 which preferably runs at a speed of between .1 and 1 r.p.m. Rotation of the vessel 44 enables distribution of the endothelial cells evenly on the inner lining of the vessel and the hydrostatic pressure head from the reservoir enables the lumen, or inner opening, of the vessel-equivalent to remain open. It should be emphasized that the above procedures are intended to be carried out asceptically.

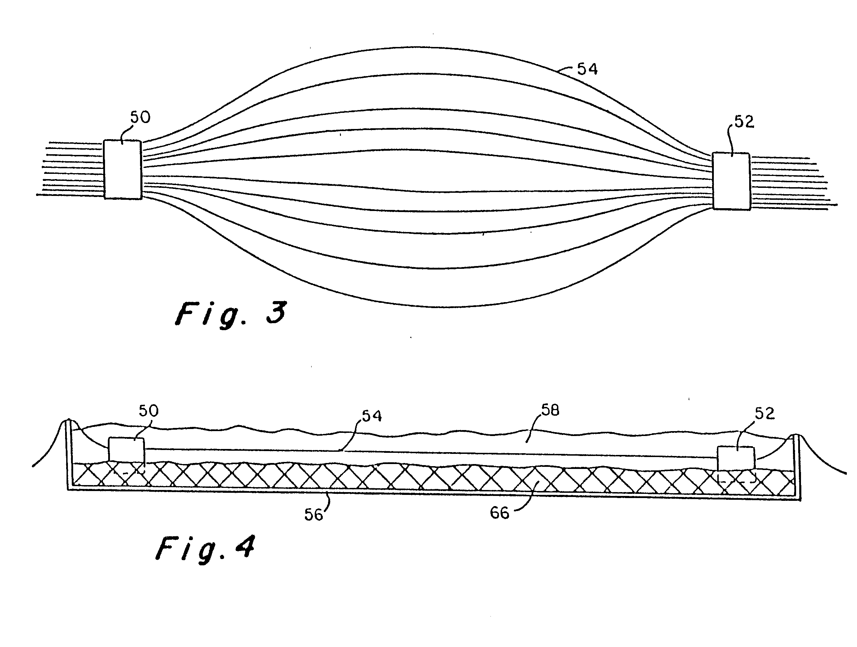

If it is desired to produce very fine vessels with small internal diameters, such as capillaries, the apparatus of Figs. 3 and 4 or Fig. 5 may be preferred since several capillaries can be fabricated in one casting procedure. The mold in Figs. 3 and 4 takes the form of a plurality of fine tubing or threads of nylon or stainless steel 54 suspended between a pair of plastic tubes or rings of dehydrated collagen 50 and 52. The threads 54 are inserted through the rings 50 and 52 and held in spaced-apart relationship by the rings. A collagen lattice with appropriate cells is cast in a pan 56 in a two-step procedure.

A first layer 66 is laid down and allowed to contract. This layer is of sufficient height to receive the threads 54 and prevent the threads from touching the bottom of the pan 56. A second layer 58 is then poured covering the threads 54. After this lattice layer has contracted, in accordance with the invention, the threads may be pulled out one at a time from either end. The plastic tube or ring 50 or 52 of dehydrated collagen, which is now free of the threads 54, is now ready to receive a pipette within which a suspension of appropriate cells is disposed. These cells are introduced into the capillaries formed in the lattice by removal of the threads and allowed to attach to the inner surfaces and culture. Fluid under slight pressure is allowed to flow through the capillaries at a slow rate to keep the channels open. As in the case of the larger vessels shown in connection with Figs. 1 and 2, after the endothelial culturing has occurred for a sufficient period of time, such as 3—5 days, the sheet of living lattice material comprising lower layer 56 and upper layer 58, may be transferred to recipient and connection made at the points of confluency of the small capillary channels left when the thread has been removed.

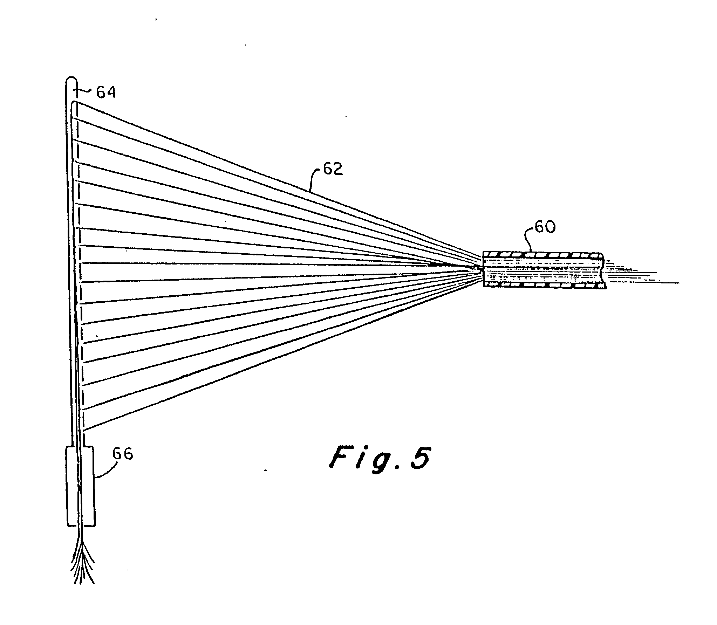

A further apparatus for casting capillaries in a slab lattice is shown in Fig. 5. In this embodiment, nylon or other threads are threaded through a threading cylinder 60, a threading tube 64 and an exit tube 66. Threading tube 64 may be formed of suitably dimensioned autoclavable plastic or glass. Cylinder 60 and exit tube 66 may be formed of dried collagen. As described in connection with Fig. 4, the assembly shown in Fig. 5 is disposed in a pan just above the bottom, so that lattice material will flow below and around it when poured. Alternatively, it may be laid into or on a freshly poured lattice. If the latter procedure is used, a second layer of lattice material may be poured over the assembly.

After a sufficient time has elapsed to allow the living lattice to gel and contract to produce a supporting structure, each thread 62 is pulled out through cylinder 66 leaving capillary channels in the lattice. When all the threads are removed a set of channels connecting, or anastomosing, at cylinder 60 will constitute a bed of capillary vessels. After all the threads are removed, threading tube 64 may be withdrawn from the lattice and a suspension of endothelial cells may be injected via the cylindrical opening at 60 into the channel. As above flow under pressure is allowed to flow through the capillaries at a slow rate to keep them open. After 5 days or less, the bed is ready for implementation, since by that time, the endothelial cells will have lined the inner channel surfaces.

Connecting tubes of dried collagen may be sewn to the severed ends of the blood vessel of the host organism from which the cells used to populate the fabricated capillary bed were taken. The connecting tubes, not shown, are then inserted into the recesses of tube 66 and tube 60 and are secured by sutures. This capillary-equivalent is then allowed to form "in vivo".

Alternatively, connecting tubes may be formed of vessel-equivalent structures produced by using the cylindrical ends of the capillary bed as the core for molding a vessel-equivalent structure on each end to serve as a connecting tube between the capillary bed-equivalent and the severed ends of the blood vessel of the host organism. Such a vessel-equivalent structure would be formed substantially as previously described in connection with Figs. 1—2.

Furthermore, as mentioned previously, it may be desirable to line the inner surface of the capillary vessels with glandular cells, such as pancreatic β cells (to boost insulin supply in the blood) or hepatocytes (liver) cells. The vessels of the capillary beds provide a large surface area through which the blood may flow. Glandular cells lining the interior surface of these vessels can provide a source of secretory products of therapeutic value.

In experiments conducted in connection with the embodiments herein described, bovine cells have been used in the process since such cells were readily available for experimentation. It is contemplated, however, that for most applications, the cells will be donated by the potential recipient of the prosthesis.

Furthermore, to more clearly approximate the natural structure of body tissue, it may be desirable to include additional constituents in the mixture used to form the lattice. Such lattice or matrix constituents as proteoglycans, glycosaminoglycans or elastin may be added to the mixture with the collagen.

**Claims**

1. A method of producing *in vitro*, a laminated collagenic tubular structure, suitable for *in vivo* use as artificial vessels and glandular prostheses, by casting collagen about a core, characterised by:

a. producing a first tubular layer by the steps of:

(1) forming a first aqueous mixture of collagen fibrils, nutrient medium and a first cellular contractile agent capable of interacting with collagen fibrils,

(2) introducing the said first mixture into an annular casting chamber,

(3) maintaining the annular casting chamber containing the said first mixture under conditions sufficient to allow a gel to form therein and to allow radial contraction of the gel with expression of aqueous liquid therefrom resulting from interaction of the cellular contractile agent with collagen fibrils thereby to form a contracted hydrated collagen lattice as the first layer of the multilayered tubular structure, and

(4) removing aqueous liquid expressed in the formation of the first layer from the annular casting chamber;

b. producing a second tubular layer outwardly of the first layer by the steps of:

(1) forming a second aqueous mixture of collagen fibrils, nutrient medium and a second cellular contractile agent capable of interacting with collagen fibrils,

(2) introducing the said second mixture into the annular casting chamber,

(3) maintaining the annular casting chamber containing the said second mixture under conditions sufficient to allow a gel to form therein and to allow radial contraction of the gel with expression of aqueous liquid therefrom resulting from interaction of the cellular contractile agent with collagen fibrils to thereby form a second contracted hydrated collagen lattice as another layer of the multi-layered tubular structure; and

c. removing the resulting living multi-layered tubular structure from the annular casting chamber.

2. A method according to claim 1, characterised by lining the living multi-layered tubular structure on its inner wall with living cells.

3. A method according to claim 2, characterised by the living cells being selected from pancreatic β cells, hepatocyte cells, and endothelial cells.

4. A method according to claim 1, 2 or 3, characterised in that the first contractile agent comprises smooth muscle cells.

5. A method according to claim 1, 2, 3 or 4, characterised in that the second contractile agent comprises fibroblast cells.

6. A method of producing, *in vitro* a laminated collagenic tubular prosthesis, suitable for *in vivo* use, by casting collagen about a core, characterised by:

a. fabricating a cylindrical smooth muscle cell layer as follows:

(aa) separately preparing (i) an aqueous acidic mixture comprising nutrient medium and collagen fibrils and (ii) a mixture of smooth muscle cells suspended in nutrient medium,

(ab) raising the pH of mixture (i) to neutrality and quickly combining mixture (i) and mixture (ii) and pouring the combined mixture into a casting chamber having an inner core member and an outer cylindrical wall structure, to form a first lattice,

(ac) incubating the first lattice for a period sufficient to enable collagen fibrils to be compacted by the cells so that aqueous liquid is expressed out of the lattice as the lattice contracts radially about the core,

(ad) removing aqueous liquid expressed in step (ac),

(ae) repeating steps (aa)—(ad) if additional layers of smooth muscle cells are desired;

b. fabricating a second layer, containing fibroblast cells, on said cylindrical smooth muscle layer(s) as follows:

(ba) separately preparing (i) an aqueous acidic mixture comprising nutrient medium and collagen fibrils and (ii) a mixture of fibroblast cells suspended in nutrient medium,

(bb) raising the pH of mixture (i) to neutrality and quickly combining mixture (i) and mixture (ii) and pouring the combined mixture into a casting chamber having as an inner core member the previously-formed cylindrical smooth muscle cell layer and an outer cylindrical wall structure, to form a second lattice,

(bc) incubating the second lattice for a period sufficient to enable collagen fibrils to be contracted by the fibroblast cells so that aqueous liquid is expressed out of the second lattice as it contracts radially about the smooth muscle layer(s) disposed internally of the second lattice, and

(bd) removing aqueous liquid expressed in step (bc); and

c. lining the inner wall of the cylindrical smooth muscle cell layer with living cells.

7. A method according to claim 6, characterised by the living cells used in step (c) are endothelial cells or glandular cells.

8. A method according to any of claims 1 to 7, further characterised by adding a reinforcing sleeve of inert material to the said living multi-layered tubular structure, the sleeve for example being a plastic mesh sleeve.

9. The method according to claim 8, characterised by locating said sleeve between the first and second tubular layers of the multi-layered structure.

10. The method according to claim 9, characterised in that the said sleeve is first slipped onto a tube and the tube is subsequently slipped over the first formed tubular structure, the tube is thereupon removed leaving the sleeve positioned over the first tubular structure and then the second tubular structure is formed about the said sleeve.

11. The method according to any of claims 8 to 10, wherein the said sleeve, which is a plastics material, is pretreated to increase its electronegativity, for example by subjecting it to plasma.

12. A method according to claim 6 or claim 7, further characterised by including a plastic mesh within said living prosthesis thereby to reinforce the prosthesis and to enhance its elasticity.

13. The method according to claim 12, characterised by the said plastic mesh comprising a sleeve, which is positioned between the layers of smooth muscle cells and fibroblast cells, said sleeve for example being made of polethylene terephthalate.

14. The method according to claim 12 or claim 13, characterised by the plastic mesh being pretreated to increase its electronegativity, for example by exposing the plastic mesh to a plasma.

15. A method of producing, *in vitro*, a collagenic structure by casting hydrated collagen about core means to form a pierced collagenic structure, suitable for *in vivo* use as a capillary bed, characterised by the steps of:

a. forming a mixture of collagen fibrils, nutrient medium and a cellular contractile agent capable of interacting with collagen fibrils to produce a contracted collagen structure;

b. introducing the said mixture into a casting chamber containing a plurality of threads therein, said threads serving as core means for defining a plurality of capillary passages in the finished collagenic structure;

c. maintaining the casting chamber under conditions which allow a gel to form around the threads and to allow radial contraction of the gel resulting from interaction of the cellular contractile agent with collagen fibrils, thereby to form a contracted hydrated collagen lattice having a plurality of threads embedded therein;

d. removing the threads from the lattice to produce a plurality of capillary channels throughout the contracted collagenic structure; and

e. lining the capillary channels on their inner surfaces with endothelial cells.

16. A method according to claim 15, characterised by preparing first and second collagen mixtures and introducing them sequentially into the casting chamber to form a cast, two-layer contracted collagen structure about the threads.

**Patentansprüche**

1. Verfahren zur In-Vitro-Herstellung einer geschichteten, rohrförmigen Kollagenstruktur zur In-Vivo-Verwendung als künstliche Gefäße und Drüsenprothesen durch Gießen von Kollagen um einen Kern, dadurch gekennzeichnet, daß

a) eine erste rohrförmige Schicht hergestellt wird, indem

(1) ein erstes wässriges Gemisch aus Kollagenfibrillen, einem Nährmedium und einem ersten Zellenkontraktionsmittel, das in der Lage ist, in Wirkverbindung zu Kollagenfibrillen zu treten, hergestellt wird,

(2) das erste Gemisch in eine ringförmige Gießkammer eingegeben wird,

(3) die das erste Gemisch enthaltende Gießkammer unter Bedingungen gehalten wird, die dazu führen, daß ein Gel in dieser gebildet wird und eine radiale Kontraktion des Gels unter Ausdrücken einer wässrigen Flüssigkeit aus diesem eintritt, die aus dem Einwirken des Zellenkontraktionsmittels auf die Kollagenfibrillen resultiert, wodurch ein kontrahiertes wasserhaltiges Kollagengitter als erste Schicht der mehrschichtigen rohrförmigen Struktur gebildet wird, und

(4) die bei der Bildung der ersten Schicht ausgedrückte wässrige Flüssigkeit aus der ringförmigen Gießkammer entfernt wird;

b) eine zweite rohrförmige Schicht außerhalb der ersten Schicht hergestellt wird, indem

(1) ein zweites wässriges Gemisch aus Kollagenfibrillen, einem Nährmedium und einem zweiten Zellenkontraktionsmittel gebildet wird, das in der Lage ist, mit Kollagenfibrillen in Wirkverbindung zu treten,

(2) das zweite Gemisch in die ringförmige Gießkammer eingeführt wird,

(3) die das zweite Gemisch enthaltende ringförmige Gießkammer unter Bedingungen gehalten wird, die dazu führen, daß ein Gel in dieser gebildet wird und eine radiale Kontraktion des Gels unter Ausdrücken einer wässrigen Flüssigkeit aus diesem eintritt, die aus dem Einwirken des Zellenkontraktionsmittel auf die Kollagenfibrillen resultiert, wodurch ein zweites kontrahiertes wasserhaltiges Kollagengitter als weitere Schicht der mehrschichtigen rohrförmigen Struktur gebildet wird; und

c) die resultierende lebende mehrschichtige rohrförmige Struktur aus der ringförmigen Gießkammer entfernt wird.

2. Verfahren nach Anspruch 1, dadurch gekennzeichnet, daß die lebende mehrschichtige rohrförmige Struktur auf ihrer Innenwand mit lebenden Zellen ausgekleidet wird.

3. Verfahren nach Anspruch 2, dadurch gekennzeichnet, daß die lebenden Zellen aus pankreatischen Zellen, Hepatozytzellen und Endothelialzellen ausgewählt werden.

4. Verfahren nach Anspruch 1, 2 oder 3, dadurch gekennzeichnet, daß das erste Kontraktionsmittel Weichmuskelzellen umfaßt.

5. Verfahren nach Anspruch 1, 2, 3 oder 4, dadurch gekennzeichnet, daß das zweite Kontraktionsmittel Fibroblastzellen umfaßt.

6. Verfahren zur In-Vitro-Herstellung einer geschichteten rohrförmigen Kollagenprothese zur In-Vivo-Verwendung durch Gießen von Kollagen um einen Kern, dadurch gekennzeichnet, daß

a) eine zylindrische Weichmuskelzellenschicht wie folgt hergestellt wird:

(aa) es werden gesondert (i) ein wässriges saures Gemisch aus Nährmedium und Kollagenfibrillen und (ii) ein Gemisch aus in einem Nährmedium suspendierten Weichmuskelzellen hergestellt,

(ab) der pH-Wert des Gemisches (i) wird auf Neutralwert angehoben, und das Gemisch (i) sowie das Gemisch (ii) werden schnell miteinander verbunden, und die Gemischverbindung wird in eine Gießkammer mit einem inneren Kernteil und einer äußeren zylindrischen Wand zur Bildung eines ersten Gitters gegossen,

(ac) das erste Gitter wird für einen Zeitraum einem Inkubationsprozeß ausgesetzt, in dem die Kollagenfibrillen von den Zellen verdichtet werden können, so daß eine wässrige Flüssigkeit aus dem Gitter bei dessen radialer Kontraktion um den Kern ausgedrückt wird,

(ad) die in dem Verfahrensschritt (ac) ausgedrückte wässrige Flüssigkeit wird entfernt,

(ae) die Schritte (aa) bis (ad) werden wiederholt, falls zusätzliche Weichmuskelzellenschichten gewünscht werden;

b) eine zweite Schicht, die Fibroblastzellen enthält, auf die zylindrische (n) Weichmuskelschicht(en) wie folgt aufgebracht wird:

(ba) es werden gesondert (i) ein wässriges saures Gemisch aus Nährmedium und Kollagenfibrillen und (ii) ein Gemisch aus in Nährmedium suspendierten Fibroblastzellen hergestellt,

(bb) der pH-Wert des Gemisches (i) wird auf Neutralwert angehoben, und das Gemisch (i) sowie das Gemisch (ii) werden schnell verbunden, und die Gemischverbindung wird in eine Gießkammer gegossen, die als Innenkernteil die zuvor geformte zylindrische Weichmuskelzellenschicht und eine äußere zylindrische Wand aufweist, um ein zweites Gitter zu bilden,

(bc) das zweite Gitter wird einem Inkubationsprozeß über einen Zeitraum ausgesetzt, in dem Kollagenfibrillen von den Fibroblastzellen kontrahiert werden können, so daß eine wässrige Flüssigkeit aus dem zweiten Gitter ausgedrückt wird, während es radial um die Weichmuskelschicht(en) kontrahiert, die innerhalb des zweiten Gitters angeordnet ist (sind), und

(bd) die im Verfahrensschritt (bc) ausgedrückte wässrige Flüssigkeit wird entfernt; und

c) die Innenwand der zylindrischen Weichmuskelzellzchicht wird mit lebenden Zellen ausgekleidet.

7. Verfahren nach Anspruch 6, dadurch gekennzeichnet, daß endotheliale Zellen oder Drüsenzellen als lebende Zellen im Verfahrensschritt (c) verwendet werden.

8. Verfahren nach einem der Ansprüche 1 bis 7, dadurch gekennzeichnet, daß eine Verstärkungshülse aus inertem Material der lebenden mehrschichtigen rohrförmigen Struktur hinzugefügt wird und daß die Hülse beispielsweise eine Kunststoffmaschenhülse ist.

9. Verfahren nach Anspruch 8, dadurch gekennzeichnet, daß die Hülse zwischen der ersten und der zweiten rohrförmigen Schicht der Mehrlagenstruktur angebracht wird.

10. Verfahren nach Anspruch 9, dadurch gekennzeichnet, daß die Hülse zunächst auf ein Rohr geschoben und das Rohr anschließend über die erste hergestellte rohrförmige Struktur geschoben wird, das Rohr sodann entfernt wird, wobei die Hülse in ihrer Position über der ersten rohrförmigen Struktur belassen wird, und sodann die zweite rohrförmige Struktur über der Hülse gebildet wird.

11. Verfahren nach einem der Ansprüche 8 bis 10, dadurch gekennzeichnet, daß die Hülse, die aus einem Kunststoffmaterial besteht, zur Erhöhung ihrer Elektronegativität vorbehandelt wird, indem sie beispielsweise einem Plasma ausgesetzt wird.

12. Verfahren nach Anspruch 6 oder 7, dadurch gekennzeichnet, daß ein Kunststoffnetz in die Lebendprothese einbezogen wird, um diese zu verstärken und ihre Elastizität zu erhöhen.

13. Verfahren nach Anspruch 12, dadurch gekennzeichnet, daß das Kunststoffnetz von einer Hülse gebildet wird, die zwischen den Lagen der Weichmuskelzellen und der Fibroblastzellen angeordnet wird, und daß die Hülse beispielsweise aus Polyäthylenterephthalat hergestellt wird.

14. Verfahren nach Anspruch 12 oder 13, dadurch gekennzeichnet, daß das Kunststoffnetz zur Erhöhung seiner Elektronegativität vorbehandelt wird, indem es beispielsweise einem Plasma ausgesetzt wird.

15. Verfahren zur In-Vitro-Herstellung einer Kollagenstruktur durch Gießen wasserhaltigen Kollagens um eine Kerneinrichtung zur Bildung einer durchbohrten Kollagenstruktur zur In-Vivo-Verwendung als Kapillarbett, dadurch gekennzeichnet, daß

a) ein Gemisch aus Kollagenfibrillen, Nährmedium und einem Zellenkontraktionsmittel gebildet wird, das in der Lage ist, eine Wirkverbindung mit Kollagenfibrillen einzugehen, um eine kontrahierte Kollagenstruktur zu erzeugen;

b) das Gemisch in eine eine Mehrzahl von Fäden enthaltende Gießkammer eingeführt wird, wobei die Fäden als Kernmittel zum Bilden einer Mehrzahl von Kapillarkanälen in der fertiggestellten Kollagenstruktur dienen;

c) die Gießkammer unter Bedingungen gehalten wird, die zu einer Gelbildung um die Fäden und zu einer radialen Kontraktion des Gels führen, die aus dem Einwirken des Zellenkontraktionsmittels auf die Kollagenfibrillen resultiert, wodurch ein kontrahiertes wasserhaltiges Kollagengitter mit einer Mehrzahl von in diesem eingebetteten Fäden gebildet wird;

d) die Fäden aus dem Gitter zur Erzeugung einer Mehrzahl von Kappillarkanälen durch die kontrahierte Kollagenstruktur entfernt werden und

e) die Kapillarkanäle auf ihren Innenflächen mit endothelialen Zellen ausgekleidet werden.

16. Verfahren nach Anspruch 15, dadurch gekennzeichnet, daß ein erstes und ein zweites Kollagengemisch hergestellt wird und diese aufeinanderfolgend in die Gießkammer zur Bildung einer gegossenen, zweilagigen, kontrahierten Kollagenstruktur um die Fäden eingeführt werden.

## Revendications

1. Procédé de fabrication in vitro d'une structure tubulaire collagénique stratifiée, propre à l'utilisation in vivo comme vaisseaux artificiels et prothèses glandulaires, par moulage de collagène autour d'un noyau, caractérisé en ce que:

a) l'on forme une première couche tubulaire par les opérations consistant:

(1) à préparer un premier mélange aqueux de fibrilles de collagène, de mileu nutritif et d'un premier agent cellulaire contractile capable d'interaction avec les fibrilles de collagène,

(2) à introduire ce premier mélange dans une chambre annulaire de moulage,

(3) à maintenir la chambre annulaire de moulage, contenant le premier mélange, dans des conditions telles qu'un gel s'y forme et qu'une contraction radiale du gel puisse s'y produire avec expression de liquide aqueux en conséquence de l'interaction de l'agent cellulaire contractile avec les fibrilles de collagène, de manière à former un réseau contracté de collagène hydraté en tant que première couche de la structure tubulaire multicouches et

(4) à enlever de la chambre annulaire de moulage le liquide aqueux exprimé au cours de la formation de la première couche;

b) l'on forme une second couche tubulaire à l'extérieur de la première couche par les opérations consistant:

(1) à préparer un second mélange aqueux de fibrilles de collagène, de milieu nutritif et d'un second agent cellulaire contractile capable d'interaction avec les fibres de collagène,

(2) à introduire ce second mélange dans la chambre annulaire de moulage,

(3) à maintenir la chambre annulaire de moulage, contenant le second mélange, dans des conditions telles qu'un gel puisse s'y former et qu'une contraction radiale du gel puisse s'y produire avec expression de liquide aqueux en conséquence de l'interaction de l'agent cellulaire contractile avec les fibrilles de collagène, de manière à former un second réseau contracté de collagène hydraté en tant qu'autre couche de la structure tubulaire multicouches; et

c) l'on retire de la chambre annulaire de moulage la structure tubulaire multi-couches vivante ainsi obtenue.

2. Procédé selon la revendication 1, caractérisé en ce qu'on revêt la structure tubulaire multicouches vivante de cellules vivantes sur sa paroi interne.

3. Procédé selon la revendication 2, caractérisé en ce que les cellules vivantes sont choisies parmi des cellules pancréatiques, des cellules hépato-cytaires et des cellules endothéliales.

4. Procédé selon l'une quelconque des revendications 1 à 3, caractérisé en ce que le premier agent contractile comprend des cellules musculaires lisses.

5. Procédé selon l'une quelconque des revendications 1 à 4, caractérisé en ce que le second agent contractile comprend des cellules fibroblastiques.

6. Procédé de fabrication in vitro d'une prothèse tubulaire collagénique stratifiée, propre à l'utilisation in vivo, par moulage de collagène autour d'un noyau, caractérisé en ce que:

a) l'on fabrique une couche cylindrique de cellules musculaires lisses en procédant de la manière suivante:

(aa) on prépare séparément (i) un mélange aqueux de caractère acide comprenant un milieu nutritif et des fibrilles de collagène et (ii) un mélange de cellules musculaires lisses en suspension dans un milieu nutritif,

(ab) on élève à la neutralité le pH du mélange (i), on combine rapidement le mélange (i) et le mélange (ii) et on verse le mélange combiné dans une chambre de moulage comportant un noyau intérieur et une paroi cylindrique extérieure, pour former un premier réseau,

(ac) on met en incubation le premier réseau pendant une période suffisante pour que les fibrilles de collagène soient comprimées par les cellules, de telle manière qu'un liquide aqueux soit exprimé du réseau, tandis que celui-ci si contracte radialement autour du noyau,

(ad) on extrait le liquide aqueux exprimé dans l'opération (ac),

(ae) on répète les opérations (aa) à (ad) si l'on veut obtenir d'autres couches de cellules musculaires lisses;

b) l'on fabrique une seconde couche, contenant des cellules fibroblastiques, sur la ou les couches cylindriques de cellules musculaires lisses en procédant de la manière suivante:

(ba) on prépare séparément (i) un mélange aqueux de caractère acide comprenant un milieu nutritif et des fibrilles de collagène et (ii) un mélange de cellules fibroblastiques en suspension dans un mélange nutritif,

(bb) on élève à la neutralité le pH du mélange (i), on combine rapidement le mélange (i) et le mélange (ii) et on verse le mélange combiné dans une chambre de moulage comportant, en tant que noyau intérieur, la couche cylindrique de cellules musculaires lisses formée précédemment et une paroi cylindrique extérieure, pour former un second réseau,

(bc) on met en incubation le second réseau pendant une période suffisante pour que les fibrilles de collagène puissent être contractées par les cellules fibroblastiques, de telle manière qu'un liquide aqueux soit exprimé du second réseau, tandis que celui-ci se contracte radialement autour de la couche ou des couches de cellules musculaires lisses disposées à l'intérieur du second réseau et

(bd) on extrait le liquide aqueux exprimé dans l'opération (bc); et

c) l'on revêt de cellules vivantes la paroi interne de la couche cylindrique de cellules musculaires lisses.

7. Procédé selon la revendication 6, caractérisé en ce que les cellules vivantes utilisées dans l'opération (c) sont des cellules endothéliales ou des cellules glandulaires.

8. Procédé selon l'une quelconque des revendications 1 à 7, caractérisé en outre en ce qu'on adjoint un manchon de renfort de matière inerte à la structure tubulaire multi-couches vivante, ce manchon étant par exemple un manchon de treillis de matière plastique.

9. Procédé selon la revendication 8, caractérisé en ce qu'on interpose le manchon entre la première et la seconde couches tubulaires de la structure multi-couches.

10. Procédé selon la revendication 9, caractérisé en ce que le manchon est tout d'abord glissé sur un tube, puis le tube est glissé sur la première structure tubulaire formée, après quoi le tube est retiré, laissant le manchon en place sur la première structure tubulaire, et la seconde structure tubulaire est ensuite formée autour du manchon.

11. Procédé selon l'une quelconque des revendications 8 à 10, dans lequel le manchon, qui est en une matière plastique, est soumis à un pré-traitement visant à augmenter son électronégativité, par exemple en l'exposant à un plasma.

12. Procédé selon la revendication 6 ou 7, caractérisé en outre par l'inclusion d'un treillis de matière plastique dans la prothèse vivante, afin de renforcer celle-ci et d'augmenter son élasticité.

13. Procédé selon la revendication 12, caractérisé en ce que le treillis de matière plastique constitue un manchon qui est interposé entre les couches de cellules musculaires lisses et de cellules fibroblastiques, ce manchon étant par exemple fait de téréphtalate de polyéthylène.

14. Procédé selon la revendication 12 ou 13, caractérisé en ce que le treillis de matière plastique est soumis à un pré-traitement visant à augmenter son électronégativité, par exemple par exposition du treillis de matière plastique à un plasma.

15. Procédé de fabrication in vitro d'une structure collagénique par moulage de collagène hydraté autour d'un noyau pour former une structure collagénique ajourée, propre à l'utilisation in vivo comme réseau capillaire, caractérisé par les opérations consistant:

a) à préparer un mélange de fibrilles de collagène, de milieu nutritif et d'un agent cellulaire contractile capable d'interaction avec les fibrilles de collagène pour produire une structure de collagène contractée;

b) à introduire ce mélange dans une chambre de moulage qui contient une multiplicité de fils, ces fils servant de noyaux pour la formation d'une multiplicité de passages capillaires dans la structure collagénique achevée;

c) à maintenir la chambre de moulage dans des conditions telles qu'un gel puisse se former autour des fils et qu'une contraction radiale du gel puisse se produire en conséquence de l'interaction de l'agent cellulaire contractile avec les fibrilles de collagène, de manière à former un réseau contracté de collagène hydraté dans lequel est noyée une multiplicité de fils;

d) à retirer les fils du réseau pour créer une multiplicité de passages capillaires à travers la structure collagénique contractée; et

e) à revêtir de cellules endothéliales les passages capillaires sur leurs surfaces internes.

16. Procédé selon la revendication 15, caractérisé en ce qu'on prépare un premier et un second mélanges de collagène et on les introduit successivement dans la chambre de moulage, pour former autour des fils une structure moulée en deux couches de collagène contracté.

*Fig. IB*

*Fig. IA*

MESH
SLEEVE, *11*

SMC

FL

*Fig. I*

Fig. 2

*Fig. 3*

*Fig.4*

0 078 314

Fig. 5

4